# EUROPEAN PATENT SPECIFICATION

(11) **EP 3 476 314 B1**
(45) Date of publication and mention of the grant of the patent: **26.07.2023**
(21) Application number: 17819142.5
(22) Date of filing: 21.06.2017
(51) Int. Cl.: A61B 17/12, A61B 17/3205, A61B 17/06, A61B 17/00

(54) **SELF-TIGHTENING ELASTIC LINE LIGATOR**
SELBSTSPANNENDER ELASTISCHER LEITUNGSLIGATOR
LIGATUREUR DE LIGNE ÉLASTIQUE AUTO-SERRANT

(30) Priority: 28.06.2016 CN 201610493057; 28.06.2016 CN 201610493124; 28.06.2016 CN 201610488738
(43) Date of publication of application: 01.05.2019
(73) Proprietor: Wellcare (WUHAN) Medical Technology Co., Ltd., Hubei 430000 (CN)
(72) Inventor: ZHANG, Hui, Wuhan, Hubei 430000 (CN)
(74) Representative: Hamer, Christopher K.
(86) International application number: PCT/CN2017/089297
(87) International publication number: WO 2018/001145

(56) References cited:
- WO-A1-2015/039488
- CN-A- 103 519 860
- CN-A- 103 519 861
- CN-A- 105 232 107
- CN-A- 105 232 107
- CN-A- 105 997 180
- CN-A- 105 997 182
- CN-A- 106 037 860
- CN-U- 206 102 690
- GB-A- 292 303
- JP-A- H10 286 224
- US-A- 5 792 151
- US-A- 5 921 993
- US-A- 6 152 936

## Description

### TECHNICAL FIELD

The present disclosure relates to medical instruments, particularly to a self-tightening elastic line ligator.

### BACKGROUND

Hemorrhoid ligation (also known as hemorrhoid apron ligation, hemorrhoid suction ligation, etc.) is a common method for treatment of hemorrhoid and has exact curative effect. The principle of the hemorrhoid ligation is ligating a special apron (e.g., rubber ring, latex ring, silicone ring, elastic line ring, etc.) to the root (or bottom) of the internal hemorrhoid to block the blood supply of the hemorrhoid by the elastic retraction force of the apron, thereby causing the hemorrhoid to become necrotic, atrophy and shedding, and achieving the purpose of healing.

The traditional instruments used for ligation are extremely simple, time-consuming, laborious and prone to complications. In order to change this situation, an automatic instrument that makes ligation surgery easy has emerged in the past decade, i.e. automatic hemorrhoid ligator (also known as continuous hemorrhoid ligator, continuous hemorrhoid suction ligator, etc.). The advantages of using such instrument for ligation surgery are that the operation is simple, fast, and accurate; a single person can complete the operation, which takes only 5 to 10 minutes; the probability of complications is low; the patient generally does not need anesthesia, and the pain is slight; and most patients do not need to be hospitalized and the cost of treatment is low.

Prior art ligator devices are disclosed in patent documents WO 2015/039488 A1, JP H10 286224 A, GB 292 303 A, CN 105 232 107 A and US 6 152 936 A.

The hemorrhoid ligators are currently available in two categories:
A: Apron hemorrhoid ligator B: Elastic line hemorrhoid ligator.
A: Apron hemorrhoid ligator:
   The apron hemorrhoid suction ligator (also known as hemorrhoid ligator) and other types of hemorrhoid ligator at home and abroad have a common feature that they all use "aprons" as the basic material for ligation of the root of the hemorrhoid, while the materials used to make the apron may include natural rubber, latex or silicone, etc.

The clinical efficacy of the hemorrhoid apron ligation is directly related to two technical indicators of "inner diameter of the apron" and "elastic retraction force of the apron". However, the use of aprons as a ligation material has some inherent disadvantages that: ① due to the inherent properties of the natural rubber (or the latex, the silicone, etc.), the inner diameter of the apron cannot be infinitely small, and generally can only be 2.0∼2.5 mm (at least not less than 1.5 mm), otherwise it will be very easy to be broken during the ligation, which means that, in the size range of 2.0∼2.5 mm (at least not less than 1.5 mm) in diameter, the ligated hemorrhoid tissue will not be subjected to any elastic retraction force, and the ulceration formed after the tissue necrosis is also about 2.0∼2.5 mm (at least not less than 1.5 mm); ② when the apron is installed to the ligator in a stretched state, it tends to gradually become fatigued as the time becomes longer, and the inner diameter becomes larger; ③ affected by factors such as climate and environment, etc., the apron is easy to age over time, which weakens the elastic retraction force.

Due to the above factors, it may lead to the following consequences that: CD apron slippage occurs in the short term after surgery, resulting in treatment failure; ② postoperative bleeding complications are caused (according to statistics, the bleeding rate after hemorrhoid apron ligation is 2%∼5%); ③ the hemorrhoid necrosis is not complete, and the ulcer surface healing is delayed, which affects the efficacy.

B: Manual elastic line hemorrhoid ligator of elastic line hemorrhoid ligator

Manual elastic line hemorrhoid suction ligator (also known as automatic elastic line ligator) can overcome the shortcoming of the apron ligator that the apron cannot be tight, but the process of fastening the target tissue is complicated, the positioning and pop-up of the push line require many operation flows, and the use is not convenient. When perform the ligation using this technical solution, the only way is releasing the ligation tissue from the emitting end first, thereafter releasing the push tube, and then manually tightening the elastic line loop of the push tube to ligate the hemorrhoid tissue (or other target tissue). During this operation, the elastic line loop is easy to slip off the tissue, thereby affecting the treatment effect. Instability of manual tensioning of the ligation loop and instability of holding during the operation may both cause the loop to slip off the tissue, thereby losing the treatment effect. Furthermore, it is difficult to control the strength for tightening the ligation loop in manual tightening. When the strength is too small, it will not be tight enough. When the strength is too large, the elastic line will be broken.

### SUMMARY

Therefore, it will be necessary to provide an automatic elastic line ligator which can automatically perform the tightening such that the size of the loop at the front end of the elastic line is rapidly reduced to a minimum or close to zero.

The invention provides a self-tightening elastic line ligator as defined by appended claim 1.

### BRIEF DESCRIPTION OF THE DRAWINGS

FIG. 1 is a schematic structural view of a self-tightening elastic line ligator of the present disclosure;
FIG. 2 is a cross-sectional view of the self-tightening elastic line ligator of the present disclosure;
FIG. 3 is a schematic structural view of the inner barrel of the present disclosure;
FIG. 4 is a schematic structural view of the limiting shaft in the misalignment mechanism of the inner and outer barrel of the present disclosure;

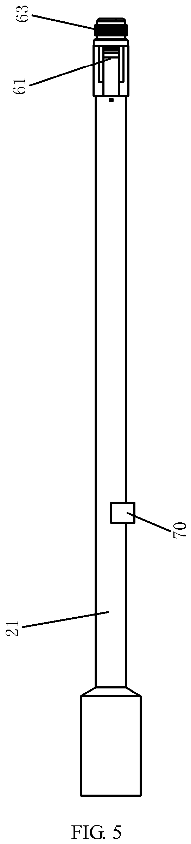

FIG. 5 is a schematic structural view of the inner tube of the body of the present disclosure;
FIG. 6 is a cross-sectional view of the body of the self-tightening elastic line ligator of the present disclosure;
FIG. 7 is a schematic structural view of the line-retracting auto-connector of the elastic line automatic tensioning mechanism of the present disclosure;
FIG. 8 is a cross-sectional view of the barrel of the self-tightening elastic line ligator of the present disclosure;
FIG. 9 is another cross-sectional view of the barrel of the self-tightening elastic line ligator of the present disclosure;
FIG. 10 is a schematic structural view of the elastic line connection end of the elastic line automatic tensioning mechanism of the present disclosure;
FIG. 11 is a schematic structural view of the elastic line connection end with the sleeve of the elastic line automatic tensioning mechanism of the present disclosure; and
FIG. 12 is a schematic structural view of the power pulling end of the elastic line automatic tensioning mechanism of the present disclosure.

### DETAILED DESCRIPTION

In order to make the objects, the technical solutions and the advantages of the present disclosure to be clearer, the present disclosure will be further described in detail below with reference to the drawings and embodiments. However, it should be understood that the specific embodiments described herein are merely illustrative and not intended to limit the present disclosure.

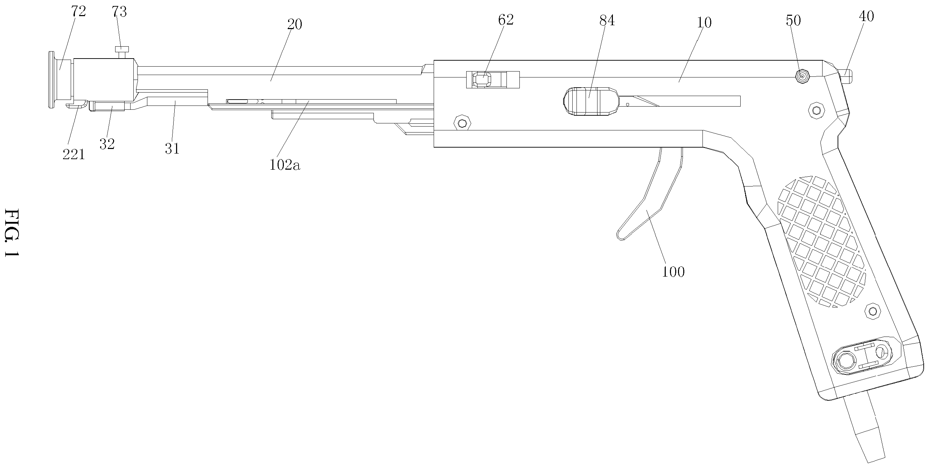

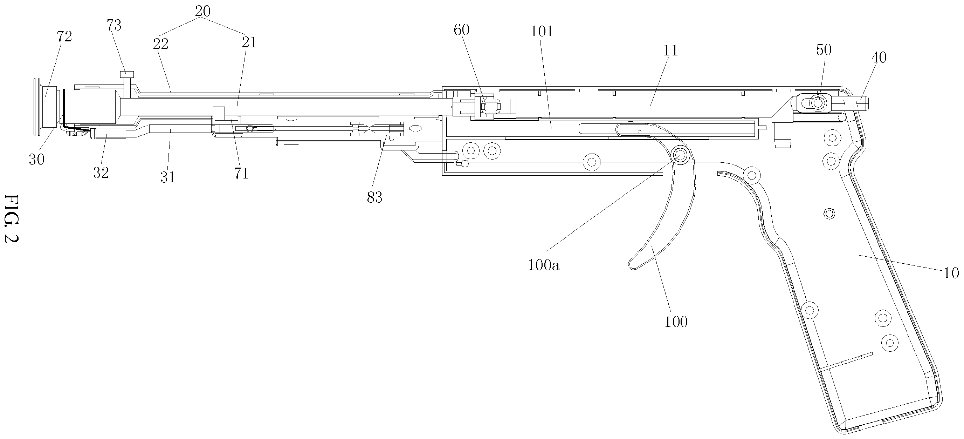

As shown in FIG. 1 and FIG. 2, the present disclosure may provide a ligator setup method for automatically tightening elastic line. The method does not form part of the claimed invention. The method may include disposing at least one elastic line 30 along an outer wall of an outer barrel 22 of the ligator. A front end of the elastic line 30 may form a size-adjustable loop loopingly disposed on an outer wall of a front end of an inner barrel 21. A tail end of the elastic line 30 may be connected to a line-retracting auto-connector 80 of the ligator. The other end of the line-retracting auto-connector 80 may be connected, via a compression spring 90, to a self-tightening trigger 100. In this case, the elastic line 30 is in a stretched state. When retracting the tail end of the elastic line 30, the self-tightening trigger 100 loosens the abutment thereof to the compression spring, and the line-retracting auto-connector 80 tightens backward the elastic line 30 under an action of a resilient force of the compression spring, thereby rapidly reducing the size of the loop at the front end of the elastic line 30.

In the present disclosure, the elastic line 30 may form the loop by a pirate knot which only allows the elastic line 20 to be pulled in one direction. The outer barrel 22 of the ligator is able to be moved with respect to the inner barrel 21 or the inner barrel 21 is able to be moved with respect to the outer barrel 22. The inner diameter of the outer barrel 22 matches the outer diameter of the inner barrel 21. In an initial state, the nozzle of the inner barrel 21 protrudes from the nozzle of the outer barrel 22. When the outer barrel 22 is moved with respect to the inner barrel 21 or the inner barrel 21 is moved with respect to the outer barrel 22, the wall of the outer barrel 22 push the elastic line 30 out of a barrel body of the inner barrel 21.

The retraction stroke of the line-retracting auto-connector 80 is set to be greater than the maximum tension length of the elastic line 30. Therefore, the power pulling end can stretch the elastic line 30 to the maximum tension length such that the diameter of the loop at the front of the elastic line 30 is reduced to a minimum diameter and close to zero, thereby achieving a more tight ligation of the target tissue. Furthermore, since the elastic line 30 has good elastic contractility, the loop of the elastic line 30 is also reduced during the process of gradually necrosis and detachment of the target tissue, until the target tissue is necrotic and detached. The ulcer surface formed thereby is extremely small, and the chance of postoperative bleeding is reduced.

The methods above may be applied in medical instruments. A self-tightening elastic line ligator using the setup methods of elastic line 30 above, as shown in FIG. 1 and FIG. 2, include a body 10 and a barrel 20. The barrel 20 includes an inner barrel 21, an outer barrel 22 and at least one elastic line 30. The elastic line 30 is disposed along an outer wall of the outer barrel 22 of the ligator. A front end of the elastic line 30 forms a size-adjustable loop loopingly disposed on an outer wall of a front end of the inner barrel 21. A tail end of the elastic line 30 is connected with a line-retracting auto-connector 80 of the ligator. The other end of the line-retracting auto-connector 80 is connected with a self-tightening trigger 100 via a compression spring. When retracting the tail end of the elastic line 30, the self-tightening trigger 100 loosens the connection thereof to the compression spring, and the line-retracting auto-connector 80 tightens backward the elastic line 30 by means of a recoiling force of the compression spring, thereby rapidly reducing the size of the loop at the front end of the elastic line 30.

The elastic line 30 includes inner and outer layers. The inner layer (elastic strip) is a strip-shaped special polymer material with high elasticity. The outer layer (wrapped layer) is a mesh braided layer, wrapped on the surface of the inner layer, also made of special polymer materials, and able to stretch with the stretching of the elastic strip of the inner layer. The special double-layer structure of the elastic line 30 not only has good elasticity, but also can withstand strong axial pulling force without breaking. When the elastic line loop in stretched state is sleeved on the target tissue, it can be further tightened due to its own elasticity to reduce its size. Furthermore, the elastic line 30 is not easy to age and fatigue when it is in a non-stretched state, therefore it has a long service life, and the ligation effect can be improved.

An inner tube 11 is disposed in the body 10. A front end of the inner tube 11 is communicated with the inner barrel 21, and the distal end of the inner tube 11 is communicated with a negative pressure air pipe, i.e., the inner barrel 21 is under negative pressure in working condition and adsorbs and retracts the target tissue. An elastic line bayonet 221 is provided at the location where the elastic line 30 is buckled on the outer barrel 22. The tail end of the elastic line 30 passes through the elastic line bayonet 221 to connect with the line-retracting auto-connector 80 of the ligator. The elastic line bayonet 221 abuts against the pirate knot of the loop. When the target tissue is absorbed and lifted up, the outer barrel 22 is moved with respect to the inner barrel 21 or the inner barrel 21 is moved with respect to the outer barrel 22, the wall of the outer barrel 22 pushes the elastic line loop out of the inner barrel 21 such that the elastic line 30 is detached from the barrel 20 and ligates the target tissue absorbed into the inner barrel 21. At the same time, relative displacement occurs between the elastic line bayonet 221 and the elastic line loop, and the elastic line bayonet 221 also pushes the elastic line 30 out of the inner barrel 21, such that the elastic line 30 is detached from the barrel 20. Furthermore, when the size of the loop is reduced, the elastic line bayonet 221 can abut against the pirate knot, thereby preventing the loop from slipping off the target tissue.

The relative displacement of the inner barrel with respect to the outer barrel is controlled by a misalignment mechanism. The misalignment mechanism is disposed at the rear end of the body, as shown in FIG. 2, and includes a reset button 40 and a misalignment button 50. The reset button 40 is coaxially connected with the distal end of the inner tube 11 of the body, and a lateral elastic member 41 for providing a resilient elastic force is provided between the reset button 40 and the body 10. Specifically, the lateral elastic member 41 provides the reset button 40 with a resilient force for backward movement. The reset button 40 is provided with a limiting hole 42. The misalignment button 50 longitudinally passes through the limiting hole 42 and is connected with the body 10 via a longitudinal elastic member 51 (not shown in the figures). The body of the misalignment button 50 is provided with a limiting shaft 52 which has a diameter matching the diameter of the limiting hole 42. The diameter of the limiting hole 42 is greater than the diameter of the body of the misalignment button 50. When the misalignment button 50 is moved downward, a misalignment occurs between the limiting shaft 52 and the limiting hole 42, such that the inner tube 11 brings the inner barrel 21 to move with respect to the outer barrel 22.

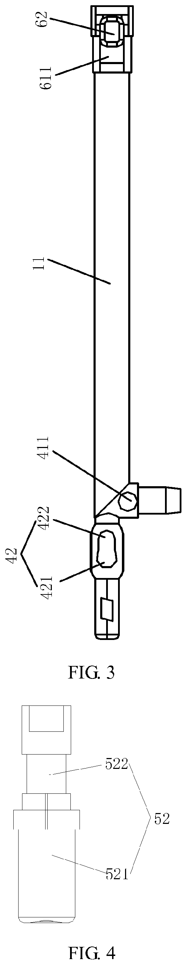

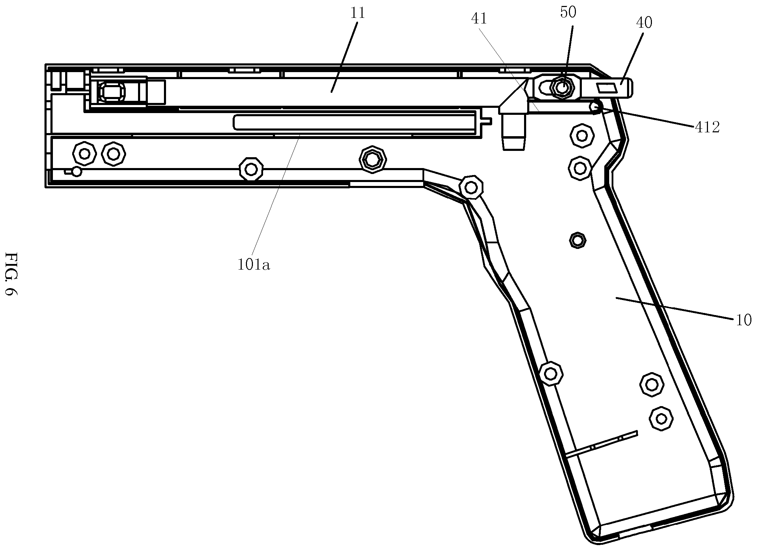

As shown in FIG. 3 and FIG. 6, the inner tube 11 is provided with a first elastic snap end 411 and the body 10 is provided with a second elastic snap end 412. The lateral elastic member 41 is disposed between the first elastic snap end 411 and the second elastic snap end 412, and the distance between the first elastic snap end 411 and the second elastic snap end 412 is greater than the free state length of the second elastic member. Therefore, the second elastic member is in an expanded state, which provides a resilient force for the relative movement between the inner tube 11 and the body 10 and, in the case that the body 10 keeps stationary, provides a resilient force for the backward movement of the reset button 40.

As shown in FIG. 3, the limiting hole 42 includes a first limiting hole 421 and a second limiting hole 422 which are communicated with each other. The diameter of the first limiting hole 421 is greater than the diameter of the second limiting hole 422, and in the lateral direction of the body 10, the second limiting hole 422 is located at front of the first limiting hole 421.

As shown in FIG. 4, the limiting shaft 52 includes a first limiting shaft 521 and a second limiting shaft 522. The diameters of the first limiting shaft 521 and the second limiting shaft 522 match the diameters of the first limiting hole 521 and the second limiting hole 422, respectively. In the longitudinal direction perpendicular to the body 10, the first limiting shaft 521 is located at inner side of the second limiting shaft 522.

When the misalignment button 50 is longitudinally moved into the body 10, the first limiting shaft 521 and the first limiting hole 421 are longitudinally misaligned. Under the action of the resilient force of the second elastic member, the second limiting shaft 522 abuts against the second limiting hole 422. At the same time, the inner tube 11 brings the inner barrel 21 to move backward with respect to the outer barrel 22. The elastic line loop sleeved on the front end of the inner barrel 21 is slid off from the inner barrel 21 under the braking of the elastic line bayonet 221 to realize the ligation of the target tissue.

When the reset button 40 is moved laterally into the body 10, the second limiting shaft 522 and the second limiting hole 422 are laterally misaligned. Under the action of the resilient force of the first elastic member, the first limiting shaft 521 is longitudinally moved outward to abut against the first limiting hole 521. At the same time, the inner tube 11 is moved forward with respect to the body 10 to the initial position so as to perform the next installation of the barrel 20.

After the elastic line loop sleeves the target tissue, the automatic tensioning mechanism for the elastic line 30 disposed on the ligator tightens backward the elastic line 30 such that the size of the loop at the front end of the elastic line 30 is rapidly reduced. The automatic tensioning mechanism for the elastic line 30 includes the line-retracting auto-connector 80 passing through the elastic line bayonet 221 and connecting with the tail end of the elastic line 30; the compression spring 90 of which one end abuts the body 10 and the other end abuts the line-retracting auto-connector 80; the self-tightening trigger 100 abutting the free end of the line-retracting auto-connector 80. When the self-tightening trigger 100 releases the abutment thereof to the free end of the line-retracting auto-connector 80, the line-retracting auto-connector 80 is rebounded backward by the compression spring 90 and causes the size of the loop at the front end of the elastic line to be rapidly reduced.

Specifically, as shown in FIG. 2, a tension stroke groove 101 is arranged in the body 10of the ligator. The line-retracting auto-connector 80 is disposed in the tension stroke groove 101. The tail end of the elastic line 30 passes through a through hole provided in an elastic line stroke groove 102 along the outer wall of the outer barrel 22 and connects with the line-retracting auto-connector 80.

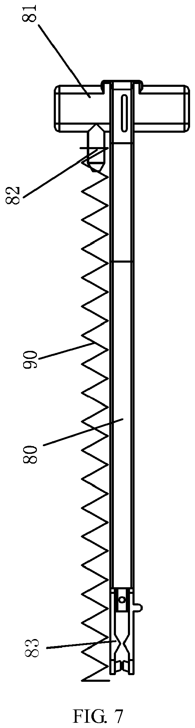

A spring groove parallel to the tension stroke groove 101 is arranged at one side of the tension stroke groove 101. The compression spring 90 is arranged in the spring groove, as shown in FIG. 7. A column 82 is laterally extended from the free end 81 of the line-retracting auto-connector 80. One end of the compression spring 90 is abutted against the spring groove, and the other end is sleeved on the column 82.

The self-tightening trigger 100 is disposed below the tension stroke groove 101. A rotation return spring is disposed on the fulcrum 100a, and provides counterclockwise resilient force for the self-tightening trigger 100. As shown in FIG. 6, the self-tightening trigger 100 is communicated with the tension stroke groove 101 via a through groove 101a. When pulling a manual self-tightening button 84 to make the free end 81 of the line-retracting auto-connector 80 to move toward the rear end of the barrel 20 to the minimum retraction stroke end of the compression spring 90, the self-tightening trigger 100 is popped counterclockwise to abut the free end 81 of the line-retracting auto-connector 80. When pressing the self-tightening trigger 100 clockwise to release the limitation to the line-retracting auto-connector 80, the line-retracting auto-connector 80 is rebounded backward under the action of the resilient force of the compression spring 90 and pulls the loop at the front end of the elastic line to reduce rapidly the size thereof. The retraction stroke of the line-retracting auto-connector 80 is greater than the maximum tension length of the elastic line 30. Therefore, the line-retracting auto-connector 80 can stretch the elastic line to the maximum tension length such that the size of the loop at the front end of the elastic line 30 is reduced to minimum and close to zero, thereby achieving more tight ligation of the target tissue.

As shown in FIG. 1, the free end 81 of the line-retracting auto-connector 80 protrudes longitudinally out of the tension stroke groove 101 from the two sides of the body 10 and forms the manual self-tightening button 84 by extension. The manual self-tightening button 84 is provided with a friction surface. Since the retraction stroke of the line-retracting auto-connector 80 is greater than the maximum tension length of the elastic line 30, when the compression spring 90 returns to the extended state there is still a stretchable distance between the free end 81 of the line-retracting auto-connector 80 and the end of the tension stroke groove 101. By arranging the friction surface on the manual self-tightening button 84 as force application point, the self-tightening button 84 is manually pulled back such that the size of the loop at the front end of the elastic line 30 continues to be reduced to the minimum size and close to zero.

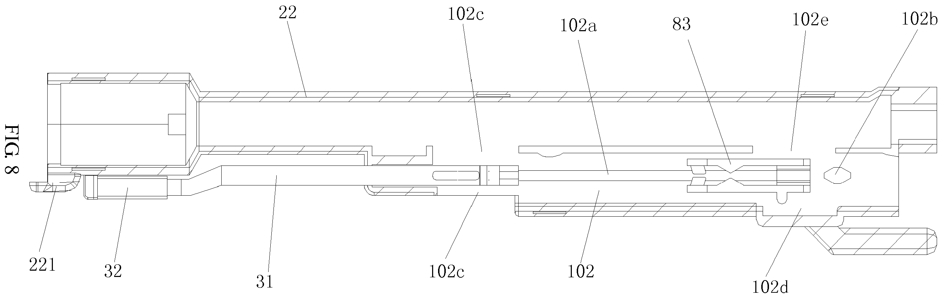

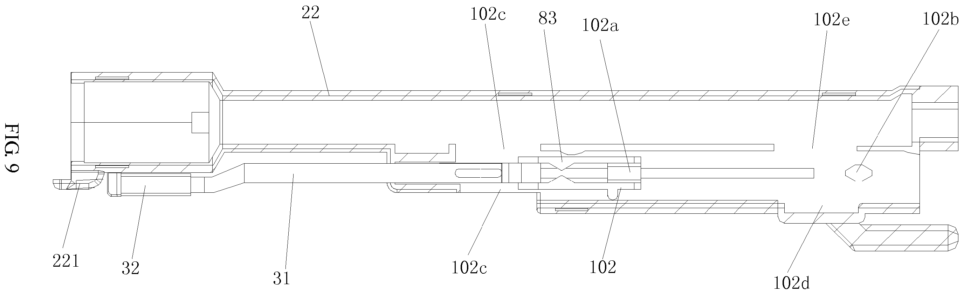

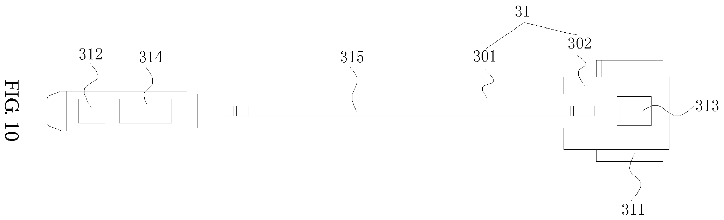

An elastic line connection end 31 is arranged at the tail end of the elastic line 30. As shown in FIG. 8, the outer wall of the outer barrel 22 of the ligator is provided with an elastic line stroke groove 102 which is connected and communicated with the tension stroke groove 101. The elastic line connection end 31 is disposed in the elastic line stroke groove 102. The elastic line connection end 31 passes through the through hole disposed in the elastic line stroke groove 102 and is tied on the tail end of the elastic line 30. The elastic line connection end 31 includes a connection end front portion 301 and a connection end rear portion 302. As shown in FIG. 8 and FIG. 9, connection end guide grooves 102a whose lengths are corresponded to the length of the elastic line stroke groove 102 are disposed on both groove walls of the elastic line stroke groove 102. As shown in FIG. 10, guide sliding wings 311 are longitudinally extended from both sides of the connection end rear portion 302. The guide sliding wings 311 are sleeved in the connection end guide groove 102a so as to ensure that the elastic line connection end 31 maintains a horizontal linear motion. The connection end front portion 301 passes through the through hole disposed in the elastic line stroke groove 102 and extends to the elastic line bayonet 221 along the outer wall of the outer barrel 22, and connects with the tail end of the elastic line 30 passing through the elastic line bayonet 221, so as to shorten the elastic tension length of the elastic line 30 as much as possible, thereby minimizing the tensile loss of the line-retracting auto-connector 80 to the elastic line loop.

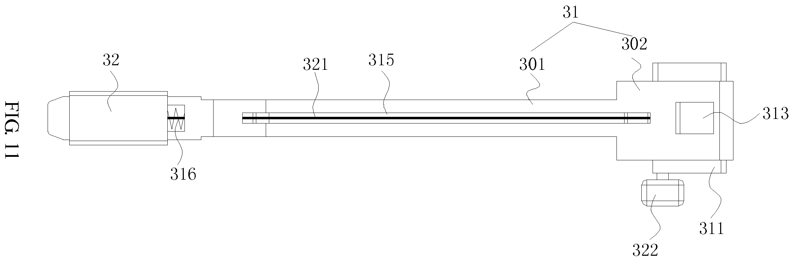

Specifically, as shown in FIG. 10 and FIG. 11, the connection end front portion 301 of the elastic line connection end 31 is provided, successively along the extension direction of the body, with a first groove 312 and a second groove 314 in which a compression spring 316 is arranged. The first groove 312 is sleeved by a sleeve 32 with a pulling cord 321. The tail end of the elastic line 30 is provided with a knot. The knot is pressed in the first groove 312 by the sleeve 32.

The connection end guide grooves 102a are communication grooves between the elastic line stroke groove 102 and the outer wall of the outer barrel 22. The body of the elastic line connection end 31 is provided with a pull guide channel 315. The pull guide channel 315 extends along the body of the elastic line connection end 31 from the second groove 314 to the guide sliding wings 311 of the connection end rear portion 302. The other end of the pulling cord 321 passes through the connection end guide groove 102a along the pull guide channel 315 and connects with a pulling member 322. The pulling member 322 abuts against the guide sliding wings 311. The rear portion of the sleeve 32 is connected with the compression spring 316 in the second groove 314. The compression spring 316 presses the sleeve 32 forward so as to prevent the sleeve 32 from accidentally falling off in non-normal use. When the elastic line is retracted and tightened, the sleeve 32 is moved backward by pulling the pulling member 322, such that the knot at the tail end of the elastic line 30 is disengaged from the first groove 312, thereby achieving the unloading of the elastic line 30.

A power pulling end 83 is disposed at one end of the line-retracting auto-connector 80 where it is connected with the elastic line 30. The tension stroke end of the power pulling end 83 corresponds to the front end of the elastic line stroke groove 102, and the retraction stroke end of the power pulling end 83 corresponds to the distal end of the elastic line stroke groove 102.

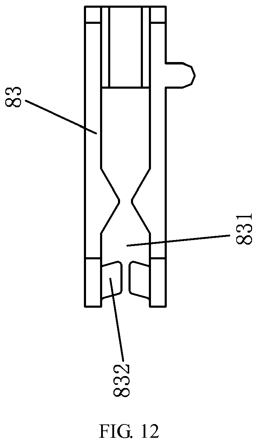

As shown in FIG. 12, the power pulling end 83 is provided with a snap opening 831 having a certain tension. The upper and lower ends of the snap opening 831 are oppositely protruded and provided with snap points 832. The snap points 832 abut against each other. As shown in FIG. 10, the connection end rear portion 302 of the elastic line connection end 31 is provided with a second holes 313 for engaging with the snap points 832.

When the power pulling end 83 makes the elastic line connection end 31 to abut against the tension stroke end of the power pulling end 83, as shown in FIG. 9, the snap opening 831 of the power pulling end 83 is opened by the elastic connection end, and the snap points 832 are snapped into the second hole 313. The power pulling end 83 is snap-connected with the elastic line connection 31. I.e., when the manual self-tightening button 84 is pulled to the minimum retraction stroke end of the compression spring 90, the line-retracting auto-connector 80 enters into the elastic line stroke groove 102. The power pulling end 83 makes the elastic line connection end 31 to abut to the tension stroke end and snap-connects the elastic line connection end 31.

As shown in FIG. 8, an expansion point 102b is disposed at the retraction stroke end of the power pulling end 83, i.e. the distal end of the elastic line stroke groove 102 is provided with an expansion point 102b, which expands the power pulling end 83 when the power pulling end 83 is pulled backward to the retraction stroke end to disengage the elastic line connection end 31 from the power pulling end 83. Preferably, the expansion point 102b is wedge-shaped, and the width of the expansion point 102b gradually increases along the retraction direction of the power pulling end 83, such that the upper and lower snap points 832 of the power pulling end 83 are outwardly extended and disengaged from the second holes 313 of the elastic line connection end 31.

The longitudinal width of the top end and distal end of the elastic line stroke groove 102 is greater than the longitudinal width of the groove body of the elastic line stroke groove 102. Specifically, as shown in FIG. 8, the front end of the elastic line stroke groove 102 is provided with upper and lower free notches 102c. The lower groove wall at the distal end of the elastic line stroke groove 102 is recessed downwardly to form an expansion groove 102d, and the upper groove wall is provided with an expansion opening 102e. The top end of the elastic line stroke groove 102 corresponds to the tension stroke end of the power pulling end 83, and the distal end of the elastic line stroke groove 102 corresponds to the retraction stroke end of the power pulling end 83. The power pulling end 83 has a longitudinal free width at the tension stroke end and the retraction stroke end.

Therefore, when the power pulling end 83 makes the elastic line connection end 31 to abut against the tension stroke end, the power pulling end 83 will not be limited by the longitudinal width of the elastic line stroke groove 102, and the snap opening 831 is longitudinally opened by the elastic connection end such that the snap points 832 are oppositely snapped into the second holes 313, thereby achieving the snap-connection of the power pulling end 83 with the elastic line connection end 31.

When the power pulling end 83 is pulled backward to the retraction stroke end, the power pulling end 83 will not be limited by the longitudinal width of the elastic line stroke groove 102, and the snap opening 831 is expanded by the expansion point 102b, such that the elastic line connection end 31 disengages from the power pulling end 83.

As shown in FIG. 2, the barrel 20 is connected with the body 10 by a snap structure 60. The snap structure 60 includes an inner buckle member 61 disposed at the rear end of the inner barrel 21 and an unbuckling member 62 disposed on the inner tube 11. The inner tube 11 is provided with a snap groove 611 cooperating with the inner buckle member 61, and the unbuckling member 62 is arranged relative to the snap groove 611. Preferably, a sealing ring 63 is arranged on the port at the distal end of the inner barrel 21 where it is connected with the inner tube 11, which is used for sealing the gap between the inner barrel 21 and the inner tube 11.

When the inner barrel 21 is snap-connected with the inner tube 11, the inner buckle member 61 is snapped in the snap groove 611, and the sealing ring 63 seals the gap between the communication ports of the inner barrel 21 and the inner tube 11 so as to ensure the negative pressure index in the inner barrel 21. When a new barrel 20 needs to be replaced, the unbuckling member 62 is pressed inwardly to push the inner buckle member 61 out of the snap groove 611 to untie the snap connection between the inner barrel 21 and the inner tube 11, thereby achieving the separation of the barrel 20 with the body 10 and facilitating the installation of the new barrel 20. The outer wall of the outer barrel 22 is also provided with a snap member, and the body 10 is provided with a bayonet groove which cooperates with the snap member. The outer barrel 22 is buckled to the body 10 by the snap member.

When it is still necessary to continue to use the elastic line 30 to perform the ligation operation after the elastic line 30 on one barrel 20 above is used, the barrel 20 whose elastic line 30 is used may be detached from the body 10 and the manual self-tightening button 84 is pulled to the minimum retraction stroke end of the compression spring 90. At this time, the self-tightening trigger 100 is popped counterclockwise to abut the line-retracting auto-connector 80. Thereafter, a new barrel 2o equipped with a new elastic line 30 is snapped on the body 10 by the snap structure 60 and the snap member. The power pulling end 83 abuts the elastic line connection end 31 to the tension stroke end and snap-connects the connection end of the elastic line 30, thereby completing the installation of the new barrel 20. Then, the ligation operation above can be restarted.

As shown in FIG. 2, an outer wall of the inner barrel 21 is provided with a limiting block 70, and an inner wall of the outer barrel 22 is recessed relatively to the limiting block 70 to form a limiting groove 71. The limiting groove 71 has a groove body extending along the length direction of the body 10. The limiting block 70 abuts in the limiting groove 71. Therefore, the inner barrel 21 is able to move with respect to the outer barrel 22 in the length direction, but maintains a fixed relative positional relationship with the outer barrel 22 in the axial direction.

The nozzle of the ligator is provided with a limiting cap 72. The limiting cap 72 is sleeved on the outer wall of the inner barrel 21 and presses the elastic line loop on the outer wall of the inner barrel 21. Further, the nozzle diameters of the outer barrel 22 and the inner barrel 21 are both greater than the diameter of the barrel body. A misalignment space is arranged between the nozzle of the outer barrel 22 and the nozzle of the inner barrel 21. A limiting bolt 73 is disposed in the misalignment space. The limiting bolt 73 passes through the wall of the outer barrel 22 and abuts the wall of the inner barrel 21. The limiting cap 72 and the limiting bolt 73 are used to ensure that the loop will not fall off the inner barrel 21 when not in use.

The present disclosure provides a ligator setup method for automatically tightening elastic line and a self-tightening elastic line ligator. They provide the backward tightening resilient elastic force for the line-retracting auto-connector 80 by the compression spring 90 and automatically tighten the elastic line 30. The doctor only needs to press the self-tightening trigger 100 to make the self-tightening trigger 100 release the abutment to the compression spring 90, and then the line-retracting auto-connector 80 can tighten backward the elastic line 30 under the action of the resilient force of the compression spring 90, thereby rapidly reducing the size of the loop at the front end of the elastic line 30 to minimum and close to zero, and achieving more tight ligation of the target tissue. Furthermore, since the elastic line 30 has good elastic contractility, the loop of the elastic line 30 is also reduced during the process of gradually necrosis and detachment of the target tissue, until the target tissue is necrotic and detached. The ulcer surface formed thereby is extremely small, and the chance of postoperative bleeding is reduced. The ligator setup methods for automatically tightening elastic line and the self-tightening elastic line ligators of the present disclosure have the advantages of simple structure, convenient operation and wide application.

The descriptions above are only the preferred embodiments of the present disclosure, and are not intended to limit the present disclosure.

## Claims

1. A self-tightening elastic line ligator, comprising a body (10), a barrel (20) and at least one elastic line (30), wherein
the barrel (20) comprises an inner barrel (21) and an outer barrel (22), the elastic line (30) is disposed along an outer wall of the outer barrel (22) of the ligator, a front end of the elastic line (30) form a size-adjustable loop loopingly disposed on an outer wall of a front end of the inner barrel (21),
the ligator further comprises an elastic line automatic tensioning mechanism, wherein the elastic line automatic tensioning mechanism comprises:
a line-retracting auto-connector (80), wherein a tail end of the elastic line (30) is connected with the line-retracting auto-connector (80),
a compression spring (90) of which one end abuts the body (10) and the other end abuts a free end (81) of the line-retracting auto-connector (80), and
a self-tightening trigger (100), which can keep abutting the free end (81) of the line-retracting auto-connector (80) with a resilient force, to keep the compression spring (90) being compressed;
wherein the elastic line automatic tensioning mechanism is configured such that when the tail end of the elastic line (30) needs to be retracted, the self-tightening trigger (100) can be pressed to release an abutment to the free end (81) of the line-retracting auto-connector (80), and thus releases an abutment to the compression spring (90), and the line-retracting auto-connector (80) is rebounded backward to tighten backward the elastic line (30) under an action of a resilient force of the compression spring (90), thereby rapidly reducing a size of the loop at the front end of the elastic line (30).

2. The self-tightening elastic line ligator of claim 1, wherein a retraction stroke of the line-retracting auto-connector (80) is greater than a maximum tension length of the elastic line (30).

3. The self-tightening elastic line ligator of claim 1, wherein an inner diameter of the outer barrel (22) matches an outer diameter of the inner barrel (21) such that when the outer barrel (22) is moved with respect to the inner barrel (21) or the inner barrel (21) is moved with respect to the outer barrel (22) a wall of the outer barrel (22) pushes the elastic line (30) out of a barrel body of the inner barrel (21).

4. The self-tightening elastic line ligator of claim 1, wherein, an elastic linebayonet (221) is provided at a location where the elastic line (30) is buckled on the outer barrel (22), and the elastic line (30) passes through the elastic line bayonet (221) to connect with the line-retracting auto-connector (80).

5. The self-tightening elastic line ligator of claim 1, wherein the barrel (20) is connected with the body (10) by a snap structure (60).

6. The self-tightening elastic line ligator of claim 1, wherein, a relative displacement of the inner barrel (21) with respect to the outer barrel (22) is controlled by a misalignment mechanism, the misalignment mechanism is disposed at a rear end of the body (10) and comprises a reset button (40) and a misalignment button (50);
the reset button (40) is coaxially connected with a distal end of an inner tube (11) of the body (10), a lateral elastic member (41) for providing a resilient elastic force is provided between the reset button (40) and the body (10), and a limiting hole (42) is disposed in the reset button (40); and
the misalignment button (50) longitudinally passes through the limiting hole (42) and connects with the body (10) via a longitudinal elastic member, and a body of the misalignment button (50) is provided with a limiting shaft (52) which has a diameter matching a diameter of the limiting hole (42);
wherein, the diameter of the limiting hole (42) is greater than a diameter of the body of the misalignment button (50), and when the misalignment button (50) is moved downward a misalignment occurs between the limiting shaft (52) and the limiting hole (42) such that the inner tube (11) brings the inner barrel (21) to move with respect to the outer barrel (22).

7. The self-tightening elastic line ligator of claim 1, wherein, a tension stroke groove (101) is arranged in the body (10), the line-retracting auto-connector (80) is disposed in the tension stroke groove (101), and the free end (81) of the line-retracting auto-connector (80) protrudes laterally out of the tension stroke groove (101) from two sides of the body (10) and forms self-tightening button (84) by extension.

8. The self-tightening elastic line ligator of claim 7, wherein, an elastic line connection end (31) is arranged at the tail end of the elastic line (30), an outer wall of the outer barrel (22) of the ligator is provided with an elastic line stroke groove (102) which is connected and communicated with the tension stroke groove (101), the elastic line connection end (31) is disposed in the elastic line stroke groove (102), the elastic line connection end (31) passes through a through hole disposed in the elastic line stroke groove (102) and is tied on the tail end of the elastic line (30), a power pulling end (83) is disposed at one end of the line-retracting auto-connector (80) where it is connected with the elastic line (30), and the power pulling end (83) is snap-connected with the elastic line connection end (31).

9. The self-tightening elastic line ligator of claim 8, wherein an expansion point is disposed at a retraction stroke end of the power pulling end (83) and used for expanding the power pulling end (83) when the power pulling end (83) is pulled backward to the retraction stroke end to disengage the elastic line connection end (31) from the power pulling end (83).

10. The self-tightening elastic line ligator of claim 9, wherein a longitudinal width of a top end and distal end of the elastic line stroke groove (102) is greater than a longitudinal width of a groove body of the elastic line stroke groove (102).

## Patentansprüche

1. Selbstspannender Gummibandligator, der einen Körper (10), eine Trommel (20) und mindestens ein Gummiband (30) umfasst, wobei die Trommel (20) eine innere Trommel (21) und eine äußere Trommel (22) umfasst, wobei das Gummiband (30) entlang einer Außenwand der äußeren Trommel (22) des Ligators angeordnet ist, wobei ein vorderes Ende des Gummibands (30) eine größenverstellbare Schlaufe bildet, die schleifenförmig an einer Außenwand eines vorderen Endes der inneren Trommel (21) angeordnet ist,
wobei der Ligator ferner einen automatischen Gummibandspannmechanismus umfasst, wobei der automatische Gummibandspannmechanismus Folgendes umfasst:
einen Bandrückzugsautoverbinder (80), wobei ein hinteres Ende des Gummibands (30) mit dem Bandrückzugsautoverbinder (80) verbunden ist,
eine Druckfeder (90), deren eines Ende am Körper (10) anliegt und deren anderes Ende an einem freien Ende (81) des Bandrückzugsautoverbinders (80) anliegt, und
einen selbstspannenden Auslöser (100), der mit einer elastischen Kraft am freien Ende (81) des Bandrückzugsautoverbinders (80) anliegen kann, um die Druckfeder (90) zusammengedrückt zu halten;
wobei der automatische Gummibandspannmechanismus so konfiguriert ist, dass, wenn das hintere Ende des Gummibands (30) zurückgezogen werden muss, der selbstspannende Auslöser (100) gedrückt werden kann, um ein Widerlager am freien Ende (81) des Bandrückzugsautoverbinders (80) zu lösen, und somit einen Widerlager an der Druckfeder (90) freigibt, und der Bandrückzugsautoverbinder (80) nach hinten zurückgezogen wird, um das Gummiband (30) unter der Wirkung einer elastischen Kraft der Druckfeder (90) nach hinten zu spannen, wodurch eine Größe der Schleife am vorderen Ende des Gummibands (30) schnell verringert wird.

2. Selbstspannender Gummibandligator nach Anspruch 1, wobei der Rückzugsweg des Bandrückzugsautoverbinders (80) größer ist als die maximale Spannungslänge des Gummibands (30).

3. Selbstspannender Gummibandligator nach Anspruch 1, wobei ein Innendurchmesser der äußeren Trommel (22) mit einem Außendurchmesser der inneren Trommel (21) übereinstimmt, so dass, wenn die äußere Trommel (22) in Bezug auf die innere Trommel (21) bewegt wird oder die innere Trommel (21) in Bezug auf die äußere Trommel (22) bewegt wird, eine Wand der äußeren Trommel (22) das Gummiband (30) aus einem Trommelkörper der inneren Trommel (21) herausdrückt.

4. Selbstspannender Gummibandligator nach Anspruch 1, wobei ein Gummiband-Bajonett (221) an einer Stelle vorgesehen ist, an der das Gummiband (30) auf der äußeren Trommel (22) geknickt ist, und das Gummiband (30) durch das Gummiband-Bajonett (221) passiert, um sich mit dem Bandrückzugsautoverbinder (80) zu verbinden.

5. Selbstspannender Gummibandligator nach Anspruch 1, wobei die Trommel (20) mit dem Körper (10) durch eine Schnappstruktur (60) verbunden ist.

6. Selbstspannender Gummibandligator nach Anspruch 1, wobei eine relative Verschiebung der inneren Trommel (21) in Bezug auf die äußere Trommel (22) durch einen Fehlausrichtungsmechanismus gesteuert wird, wobei der Fehlausrichtungsmechanismus an einem hinteren Ende des Körpers (10) angeordnet ist und einen Rückstellknopf (40) und einen Fehlausrichtungsknopf (50) umfasst;
der Rückstellknopf (40) koaxial mit einem distalen Ende eines Innenrohrs (11) des Körpers (10) verbunden ist, ein seitliches elastisches Element (41) zum Bereitstellen einer elastischen Federkraft zwischen dem Rückstellknopf (40) und dem Körper (10) vorgesehen ist, und ein Begrenzungsloch (42) im Rückstellknopf (40) angeordnet ist; und
der Fehlausrichtungsknopf (50) longitudinal durch das Begrenzungsloch (42) verläuft und mit dem Körper (10) über ein longitudinales elastisches Element verbunden ist, und ein Körper des Fehlausrichtungsknopfs (50) mit einem Begrenzungsschaft (52) versehen ist, dessen Durchmesser mit einem Durchmesser des Begrenzungslochs (42) übereinstimmt;
wobei der Durchmesser des Begrenzungslochs (42) größer als ein Durchmesser des Körpers des Fehlausrichtungsknopfs (50) ist, und wenn der Fehlausrichtungsknopf (50) nach unten bewegt wird, eine Fehlausrichtung zwischen der Begrenzungswelle (52) und dem Begrenzungsloch (42) auftritt, so dass das Innenrohr (11) die innere Trommel (21) veranlasst, sich in Bezug auf die äußere Trommel (22) zu bewegen.

7. Selbstspannender Gummibandligator nach Anspruch 1, wobei eine Spannhubnut (101) in dem Körper (10) angeordnet ist, der Bandrückzugsautoverbinder (80) in der Spannhubnut (101) angeordnet ist und das freie Ende (81) des Bandrückzugsautoverbinders (80) seitlich aus der Spannhubnut (101) von zwei Seiten des Körpers (10) herausragt und einen selbsteinziehenden Knopf (84) durch Verlängerung bildet.

8. Selbstspannender Gummibandligator nach Anspruch 7, wobei ein Gummibandverbindungsende (31) am hinteren Ende des Gummibands (30) angeordnet ist, eine Außenwand der äußeren Trommel (22) des Ligators mit einer Gummibandhubnut (102) versehen ist, die mit der Spannhubnut (101) verbunden und in Kommunikation ist, und das Gummibandverbindungsende (31) in der Gummibandhubnut (102) angeordnet ist, das Gummibandverbindungsende (31) durch ein in der Gummibandhubnut (102) angeordnetes Durchgangsloch verläuft und am hinteren Ende des Gummibands (30) befestigt ist, ein Zugkraftende (83) an einem Ende des Bandrückzugsautoverbinders (80) angeordnet ist, wo es mit dem Gummiband (30) verbunden ist, und das Zugkraftende (83) mit dem Gummibandverbindungsende (31) eingerastet ist.

9. Selbstspannender Gummibandligator nach Anspruch 8, wobei ein Expansionspunkt an einem Rückzugshubende des Zugkraftendes (83) angeordnet ist und zum Expandieren des Zugkraftendes (83) verwendet wird, wenn das Zugkraftende (83) nach hinten zum Rückzugshubende gezogen wird, um das Gummibandverbindungsende (31) vom Zugkraftende (83) zu lösen.

10. Selbstspannender Gummibandligator nach Anspruch 9, wobei eine Längsbreite eines oberen Endes und eines distalen Endes der Gummibandhubnut (102) größer ist als eine Längsbreite eines Nutkörpers der Gummibandhubnut (102) .

## Revendications

1. Dispositif de ligature par ligne élastique à serrage automatique, comprenant un corps (10), un cylindre (20) et au moins une ligne élastique (30), dans lequel le cylindre (20) comprend un cylindre interne (21) et un cylindre externe (22), la ligne élastique (30) est disposée le long d'une paroi externe du cylindre externe (22) du dispositif de ligature, une extrémité avant de la ligne élastique (30) forme une boucle à taille réglable disposée en boucle sur une paroi externe d'une extrémité avant du cylindre interne (21),
le dispositif de ligature comprend en outre un mécanisme automatique de tension de ligne élastique, dans lequel le mécanisme automatique de tension de ligne élastique comprend :
un connecteur automatique de rétraction de ligne (80), dans lequel une extrémité arrière de la ligne élastique (30) est connectée au connecteur automatique de rétraction de ligne (80),
un ressort de compression (90) dont une extrémité vient en butée contre le corps (10) et l'autre extrémité vient en butée contre une extrémité libre (81) du connecteur automatique de rétraction de ligne (80), et
une gâchette de serrage automatique (100), qui peut être maintenue en butée contre l'extrémité libre (81) du connecteur automatique de rétraction de ligne (80) avec une force élastique, pour maintenir le ressort de compression (90) comprimé ;
dans lequel le mécanisme automatique de tension de ligne élastique est configuré de sorte que quand l'extrémité arrière de la ligne élastique (30) a besoin d'être rétractée, la gâchette de serrage automatique (100) peut être pressée pour libérer une butée contre l'extrémité libre (81) du connecteur automatique de rétraction de ligne (80), et libérer ainsi une butée contre le ressort de compression (90), et le connecteur automatique de rétraction de ligne (80) est amené à rebondir vers l'arrière pour serrer la ligne élastique (30) vers l'arrière sous l'action d'une force élastique du ressort de compression (90), réduisant ainsi rapidement par ce moyen une dimension de la boucle au niveau de l'extrémité avant de la ligne élastique (30).

2. Dispositif de ligature par ligne élastique à serrage automatique selon la revendication 1, dans lequel une course de rétraction du connecteur automatique de rétraction de ligne (80) est supérieure à une longueur de tension maximale de la ligne élastique (30).

3. Dispositif de ligature par ligne élastique à serrage automatique selon la revendication 1, dans lequel un diamètre interne du cylindre externe (22) correspond à un diamètre externe du cylindre interne (21) de sorte que quand le cylindre externe (22) est déplacé par rapport au cylindre interne (21) ou le cylindre interne (21) est déplacé par rapport au cylindre externe (22), une paroi du cylindre externe (22) pousse la ligne élastique (30) hors d'un corps de cylindre du cylindre interne (21).

4. Dispositif de ligature par ligne élastique à serrage automatique selon la revendication 1, dans lequel, une baïonnette de ligne élastique (221) est fournie au niveau d'un emplacement où la ligne élastique (30) est bouclée sur le cylindre externe (22), et la ligne élastique (30) passe à travers la baïonnette de ligne élastique (221) pour se connecter au connecteur automatique de rétraction de ligne (80).

5. Dispositif de ligature par ligne élastique à serrage automatique selon la revendication 1, dans lequel le cylindre (20) est connecté au corps (10) par une structure à encliquetage (60).

6. Dispositif de ligature par ligne élastique à serrage automatique selon la revendication 1, dans lequel, un déplacement relatif du cylindre interne (21) par rapport au cylindre externe (22) est commandé par un mécanisme de désalignement, le mécanisme de désalignement est disposé au niveau d'une extrémité arrière du corps (10) et comprend un bouton de réarmement (40) et un bouton de désalignement (50) ;
le bouton de réarmement (40) est connecté de manière coaxiale avec une extrémité distale d'un tube interne (11) du corps (10), un élément élastique latéral (41) destiné à fournir une force élastique de résilience est fourni entre le bouton de réarmement (40) et le corps (10), et un orifice de limitation (42) est disposé dans le bouton de réarmement (40) ; et
le bouton de réarmement (50) passe de manière longitudinale à travers l'orifice de limitation (42) et se connecte au corps (10) via un élément élastique longitudinal, et un corps du bouton de désalignement (50) est pourvu d'un arbre de limitation (52) qui a un diamètre qui correspond à un diamètre de l'orifice de limitation (42) ;
dans lequel, le diamètre de l'orifice de limitation (42) est supérieur à un diamètre du corps du bouton de désalignement (50), et quand le bouton de désalignement (50) est déplacé vers le bas, un désalignement a lieu antre l'arbre de limitation (52) et le trou de limitation (42) de sorte que le tube interne (11) amène le cylindre interne (21) à se déplacer par rapport au cylindre externe (22).

7. Dispositif de ligature par ligne élastique à serrage automatique selon la revendication 1, dans lequel, une rainure de course de tension (101) est agencée dans le corps (10), le connecteur automatique de rétraction de ligne (80) est disposé dans la rainure de course de tension (101), et l'extrémité libre (81) du connecteur automatique de rétraction de ligne (80) fait saillie de manière latérale hors de la rainure de course de tension (101) depuis deux côtés du corps (10) et forme un bouton de serrage automatique (84) par extension.

8. Dispositif de ligature par ligne élastique à serrage automatique selon la revendication 7, dans lequel, une extrémité de connexion de ligne élastique (31) est agencée au niveau de l'extrémité arrière de la ligne élastique (30), une paroi externe du cylindre externe (22) du dispositif de ligature est pourvue d'une rainure de course de ligne élastique (102) qui est connectée et amenée à communiquer avec la rainure de course de tension (101), l'extrémité de connexion de ligne élastique (31) est disposée dans la rainure de course de ligne élastique (102), l'extrémité de connexion de ligne élastique (31) passe à travers un trou traversant disposé dans la rainure de course de ligne élastique (102) et est nouée sur l'extrémité arrière de la ligne élastique (30), une extrémité de traction de puissance (83) est disposée au niveau d'une extrémité du connecteur automatique de rétraction de ligne (80) où elle est connectée à la ligne élastique (30), et l'extrémité de traction de puissance (83) est connectée par encliquetage à l'extrémité de connexion de ligne élastique connexion (31).

9. Dispositif de ligature par ligne élastique à serrage automatique selon la revendication 8, dans lequel un point de déploiement est disposé au niveau d'une extrémité de course de rétraction de l'extrémité de traction de puissance (83) et utilisé pour déployer l'extrémité de traction de puissance (83) quand l'extrémité de traction de puissance (83) est tirée vers l'arrière vers l'extrémité de course de rétraction pour dégager l'extrémité de connexion de ligne élastique (31) de l'extrémité de traction de puissance (83).

10. Dispositif de ligature par ligne élastique à serrage automatique selon la revendication 9, dans lequel une largeur longitudinale d'une extrémité supérieure et d'une extrémité distale de la rainure de course de ligne élastique (102) est supérieure à une largeur longitudinale d'un corps de rainure de la rainure de course de la ligne élastique (102).
